Europäisches Patentamt

European Patent Office

Office européen des brevets

(19)

(11) Veröffentlichungsnummer : **0 053 293**
**B1**

(12)        EUROPÄISCHE PATENTSCHRIFT

(45) Veröffentlichungstag der Patentschrift :
15.08.84

(21) Anmeldenummer : 81109321.0

(22) Anmeldetag : 30.10.81

(51) Int. Cl.³ : **C 07 C 13/20, C 07 C 13/263,**
**C 07 C 2/50, C 07 C 2/44,**
**C 07 C 7/20**

(54) Verfahren zur thermischen Dimerisierung von Butadien.

(30) Priorität : 01.12.80 DE 3045229

(43) Veröffentlichungstag der Anmeldung :
09.06.82 Patentblatt 82/23

(45) Bekanntmachung des Hinweises auf die Patenterteilung : 15.08.84 Patentblatt 84/33

(84) Benannte Vertragsstaaten :
AT BE DE FR GB IT NL SE

(56) Entgegenhaltungen :
AU-D- 2 568 067
DD-A- 123 082
DE-A- 805 301
DE-B- 1 119 260
GB-A- 971 976
US-A- 3 951 754

(73) Patentinhaber : DEUTSCHE TEXACO AKTIENGE-
SELLSCHAFT
Überseering 40
D-2000 Hamburg 60 (DE)

(72) Erfinder : Gluzek, Karl-Heinz, Dr.
Die Schraag 11
D-4234 Alpen (DE)
Erfinder : Petersen, Olaf, Dr.
Comeniusstrasse 1
D-4005 Meerbusch 2 (DE)
Erfinder : Neier, Wilhelm, Dr.
An der Landwehr 40
D-4134 Rheinberg 3 (DE)
Erfinder : Strehlke, Günter, Dr.
Schlotweg 4
D-4100 Duisberg 74 (DE)
Erfinder : Heinemann, Karl-Heinz, Dr.
Gartenstrasse 47
D-4133 Neukirchen-Vluyn (DE)

(74) Vertreter : Müller, Hans-Jürgen et al
Müller, Schupfner & Gauger Karlstrasse 5
D-2110 Buchholz/Nordheide (DE)

## Beschreibung

Die vorliegende Erfindung bezieht sich auf ein Verfahren zur thermischen Dimerisierung von Butadien.

Verfahren zur thermischen Dimerisierung von konjugierten Diolefinen sind bekannt. Insbesondere bei der thermischen Dimerisierung von Butadien werden jedoch unerwünschte hochmolekulare Polymere gebildet, die wegen ihres Aussehens « Popcorn »-Polymere genannt werden. Es werden sogenannte « Keime » gebildet, die als Angriffsstellen für die weitere Polymerisation wirksam sind. Diese « Keime » setzen sich vornehmlich an den Wandungen der Reaktionsapparaturen fest, so daß diese von außen nach innen zuwachsen.

Da das « Popcorn »-Polymerisat in den gebräuchlichen Lösungsmitteln unlöslich ist, ist es unmöglich, es auszuwaschen. Wegen des starken Haftvermögens und der zum Teil klebrigen Konsistenz ist die mechanische Entfernung außerordentlich erschwert und in Rohrleitungen technischer Anlagen unmöglich. Auch wenn die Apparaturen mit größter Sorgfalt gereinigt wurden, verbleiben restliche Teilchen, die das unerwünschte Polymerenwachstum begünstigen. Die « Popcorn »-Polymerisation verursacht somit einen Verlust an wertvollem Rohstoff und steigert erheblich die Gesamtkosten der Produktion durch Verstopfungen von Reaktionsapparaturen und -leitungen.

Zur Vermeidung dieser unerwünschten Polymerisation ist bereits vorgeschlagen worden, in einem organischen Lösungsmittel zu arbeiten. So wird in der US-Patentschrift 2 411 822 Kerosin als Lösungsmittel vorgeschlagen. In der US-Patentschrift 3 454 665 wird ein paraffinisches Waschöl mit einem Siedepunkt von etwa 215 °C empfohlen. Die Menge des hierbei gebildeten löslichen Polymerisats ist aber beträchtlich, wenn auch Angaben über « Popcorn »-Polymerisat in diesen Patentschriften fehlen.

Gemäß Industrial and Engineering Chem., Vol. 39, Nr 7, Seiten 830-837, werden gasförmiges Stickstoffdioxyd und wäßrige Natriumnitritlösungen als allgemeine Polymerisationsinhibitoren verwendet. Diese Veröffentlichung aus dem Jahre 1947 bezieht sich auf die Vermeidung der Polymerisation von Styrol und Butadien in Lagertanks und Anlagen, die dem Reaktor zur Styrolsynthese nachgeschaltet sind. Die wäßrige Lösung von Natriumnitrit wird insbesondere als Inhibitor für die Styrolpolymerisation und als anorganisches Antioxydans ausgewiesen. Seine Wirksamkeit wurde jeweils bei 55 °C getestet, und es wurde festgestellt, daß aufgrund seiner inhibierenden Wirkung bei dieser Temperatur Styrol noch nach 100 bis 200 Tagen flüssig war. Bei einer für solche Anlagen sehr hohen Temperatur von 100 °C war Styrol nur noch 14 Tage flüssig. Die Verwendung von wäßriger Natriumnitritlösung für Polymerisationsverfahren selbst zur Unterdrückung der « Popcorn »-Bildung während der Polymerisation ist dieser Veröffentlichung nicht zu entnehmen. Dies ist auch verständlich, da die Polymerisationsverfahren selbst, insbesondere die thermischen Polymerisationsverfahren bei wesentlich höheren Temperaturen durchgeführt werden.

Zur Verhinderung der « Popcorn »-Polymerisation bei der thermischen Dimerisierung von konjugierten Olefinen sind die verschiedensten Inhibitoren vorgeschlagen worden. In der US-Patentschrift 2 943 117 wird ein aromatisches Lösungsmittel, z. B. Benzol, in Kombination mit einer wäßrigen Lösung eines Diamins oder mit wäßriger Ammoniaklösung vorgeschlagen. Nach diesen Verfahren können hochmolekulare Polymere aber nicht gänzlich unterdrückt werden.

In den deutschen Auslegeschriften 20 38 311 und 21 15 858 wird die Verwendung von N-substituierten N-Nitrosohydroxylaminen vorgeschlagen. Diese Systeme sind jedoch teuer und nur für Temperaturen bis 140 °C geeignet.

Gemäß DE-OS 18 16 826 aus dem Jahres 1968 wird die Neigung von Butadien zur Polymerisation in Lösungen dadurch vermindert, daß als Polymerisationsinhibitoren aromatische Nitroverbindungen zugegeben werden. Auf Seite 2, unterer Absatz, dieser Offenlegungsschrift heißt es wörtlich, daß bei Zimmertemperatur oder bei niedrigerer Temperatur die Polymerisation von Butadien in gewissem Umfang dadurch verhindert werden kann, daß konventionnele, bekannte Polymerisationsinhibitoren, z. B. Hydrochinon, 4-tert.-Butyl-catechol, β-Naphthylamin, Methylenblau, Natriumnitrit usw. zugegeben werden :

« Die vorgenannten Polymerisationsinhibitoren haben jedoch keine zufriedenstellende Wirkung hinsichtlich der Verhinderung der Polymerisation von Butadien, wenn die butadien-haltige Lösung einer Hitzebehandlung bei verhältnismäßig hohen Temperaturen, beispielsweise 80 bis 160 °C oder sogar bei noch höheren Temperaturen über einen längeren Zeitraum ausgesetzt werden. »

Somit ist aus dieser Offenlegungsschrift zu entnehmen, daß Natriumnitrit bei den hier in Anwendung kommenden Temperaturen als Inhibitor für « Popcorn »-Bildung nicht geeignet ist.

Aus der deutschen OS 1 805 301 und der britischen Patentschrift 971 976 geht zwar hervor, daß Nitrite als wirksame Mittel zur Verhinderung der Bildung von Popcorn-Polymeren befunden wurden, daß sie sich aber zu einer wirtschaftlichen Verwendung nicht eignen. Gemäß diesen Druckschriften wird der Einsatz von Anilin-N-oxiden bzw. Alkylhydroxylaminen empfohlen. Im übrigen beziehen sich diese vorbekannten Verfahren nicht auf die Dimerisierung von Butadien sondern auf die Wiedergewinnung von olefinischen Monomeren.

Gleichfalls bezieht sich die US-Patentschrift 3 951 754 auf ein Verfahren zur Isolierung von Butadien oder Isopren, wobei die Popcorn-Polymerisation durch Einsatz eines Inhibitors, der Natriumnitrit sein kann, verhindert werden soll. Durch diese Patentschrift ist kein kontinuierliches Verfahren zur Dimerisierung von Butadien bekannt geworden. Die in der Druckschrift beschriebenen Experimente wurden in Gegenwart von Eisenspänen, die mit Nitritlösung beaufschlagt waren, durchgeführt, wobei stets eine zumindestens geringe Polymerbildung stattfand. Ferner war nach dem vorbekannten Verfahren ein polares Lösungsmittel für Nitrit und Butadien bzw. Isopren anwesend, und die verwendeten Temperaturen überstiegen nicht 160 °C.

Gemäß der australischen Patentschrift 25 680/67 ist ein Verfahren bekannt geworden, das, ausgehend von 1,3-Butadien, bei hoher Temperatur zu 4-Vinylcyclohexen führt, wobei aber kein Nitrit als Inhibitor für Popcorn-Polymerisation eingesetzt wird.

Erfindungsgemäß wurde nun überraschenderweise gefunden, daß wäßrige Alkalinitritlösung auch bei höheren Temperaturen die « Popcorn »-Bildung zu inhibieren vermögen, ohne daß gleichzeitig die angestrebte Dimerisierung beeinträchtigt wird. Es ist hierbei zu beachten, daß einerseits für eine hinreichende Kontaktierung, Benetzung der Anlagenteile mit der wäßrigen Alkalinitritlösung gesorgt sein muß, da nur so der Inhibitor seine Wirksamkeit voll entfalten kann. Es ist vorteilhaft, das zu dimerisierende Ausgangsmaterial und die Alkalinitritlösung im Dimerisationsreaktor im Gegenstrom gegeneinander zu führen, wobei die Alkalinitritlösung am Kopf des Reaktors eingespeist wird.

Andererseits ist es von Bedeutung, weitgehend Sauerstoff und Peroxo verbindungen auszuschließen, wie dies bei allen Butadienverarbeitungsanlagen üblich ist. Gemäß einer bevorzugten Ausführungsform des Verfahrens der vorliegenden Erfindung wird das Butadienmaterial vor dem Einsatz frisch destilliert und insbesondere noch zusätzlich mit Wasserstoff in Gegenwart eines Hydrierkatalysators behandelt. Geeignet hierfür sind jegliche hydrieraktiven Katalysatoren, insbesondere Trägerkatalysatoren. Bevorzugt werden übliche leicht verfügbare Platin- und/oder Palladiumkatalysatoren, etwa auf einem $Al_2O_3$- oder $SiO_2$-Träger, eingesetzt.

Das Verfahren der vorliegenden Erfindung ist durch Anspruch 1 definiert. Es ist besonders überraschend, daß eine wäßrige Alkalinitrilösung für die thermische Dimerisierung zur Unterdrückung der « Popcorn »-Bildung eingesetzt werden kann, da u. a. aus DE-OS 18 16 826 zu entnehmen ist, daß Natriumnitrit bei den hier zur Anwendung kommenden Temperaturen als Inhibitor für die « Popcorn »-Bildung nicht geeignet ist, siehe die Tabellen der Beispiele 2, 4, 5 und 7 dieser Offenlegungsschrift, jeweils Zeile 2. Entsprechende Angaben finden sich auch in US-PS 3 407 240.

Als Butadienquelle kann käufliches Reinbutadien als auch technisches Pyrolyse-$C_4$ dienen. Es ist bekannt, daß technische Pyrolyse-$C_4$-Schnitte und $C_4$-Kohlenwasserstoffgemische aufgrund von Undichtigkeiten und unzulänglicher Stickstoffspülung von Apparaturen oder Behältern für die Lagerung und den Transport sowie bei Störungen in der Stufe zur Erzeugung von $C_4$-Schnitten als auch aufgrund der Sauerstoffinitiierung bei der Herstellung von Polybutadien oder Butadien-Styrol-Copolymerisaten geringe Mengen Sauerstoff enthalten können. Der Sauerstoffgehalt solcher $C_4$-Schnitte kann 0,000 1 bis über 1 Gew.% betragen und ist damit ein Störfaktor, der entfernt werden muß.

Übliche Inhibitoren, die zur Verbesserung der Lagerstabilität des Diolefins im Ausgangsmaterial enthalten sind, stören nicht, da sie — wie bereits erwähnt — bei den hier anzuwendenden hohen Temperaturen unwirksam werden. Sie können daher im Einsatzgas verbleiben.

Aus wirtschftlichen Gründen wird als Alkalinitrit Natriumnitrit bevorzugt, jedoch sind auch andere Nitrite wie z. B. Kaliumnitrit geeignet. Die Konzentration an Alkalinitrit in der wäßrigen Lösung ist nicht kritisch und liegt im Bereich von z. B. 4-40 Gew.% ; bevorzugt wird eine ca. 7 %ige wäßrige Lösung von Natriumnitrit eingesetzt.

Für die Dimerisierungsreaktion sind hohe Temperaturen vorteilhaft, wobei im allgemeinen bei Temperaturen zwischen 180-260 °C, insbesondere zwischen 200-240 °C gearbeitet wird. Bei niedrigeren Temperaturen sinkt die Ausbeute. Beim Einsatz von Pyrolyse-$C_4$ als Frischgas wird eine Temperatur von etwa 230-250 °C bevorzugt.

Druckerhöhung begünstigt die Cyclisierungsreaktion. Die obere Druckgrenze ist durch die Konstruktionsmerkmale der Reaktionsapparatur gegeben. Gute Umsätze werden bereits bei Drücken um 100 bar erreicht.

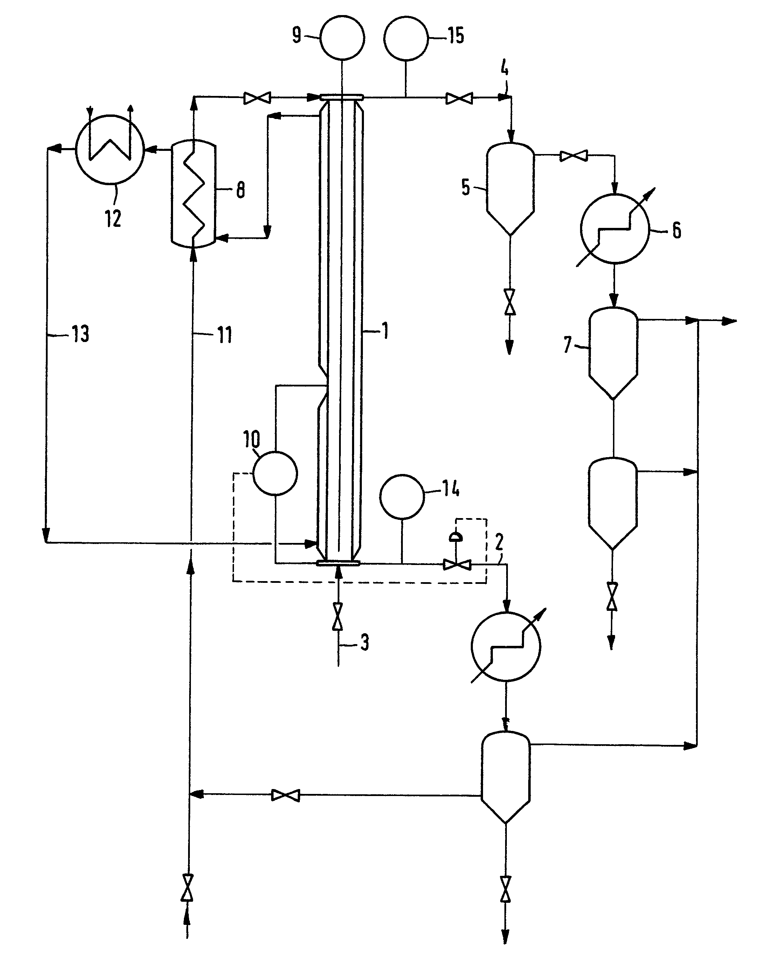

Die kontinuierliche Durchführung der thermischen Dimerisierung gemäß der vorliegenden Erfindung erfolgt zweckmäßigkeitshalber in einem Gegenstromreaktor. Die beigefügte Zeichnung erläutert die Vorrichtung zur Durchführung des Verfahrens. Der Reaktor besteht aus einem senkrecht stehenden Rohr 1 mit Ummantelung für das Wärmeübertragungsöl, das im Kreislauf 13 über Wärmeaustauscher 12 geführt wird, mit seitlichem Niveauabzug 2 und Flüssigkeitsniveauhalter 10 am unteren Reaktordrittel und mit einer zentral angeordneten Tasche für ein verschiebbares Thermoelement 9. Das Frischgas (z. B. technisches Pyrolyse-$C_4$) wird mittels einer Hochdruckpumpe (nicht gezeigt) am Boden des Reaktors durch Leitung 3 eingespeist, durchströmt die bis zum Niveauabzug 2 anstehende Inhibitorlösung (z. B. 6-7 Gew.% Natriumnitrit) und verläßt zusammen mit dem gebildeten Dimerisat und dem verdampfenden Wasser als Restgas den Reaktor am Kopf über Leitung 4. Mittels eines Hochdruckwasserabscheiders 5 wird das ammoniakhaltige Kopfwasser abgetrennt, gemessen und verworfen. Das organische Kopfprodukt wird auf Atmosphärendruck entspannt, nach Durchströmung des Kühlers 6 im Gas-Flüssigkeitsscheiders 7 in Gas- (Restgas) und Flüssigphase (Dimerisat) aufgetrennt und bilanziert. Manometer 14, 15 sind in Leitungen 2 und 4 zur Erkennung von Druckfifferenzen vorgesehen.

Die Inhibitorlösung wird der aufsteigenden organischen Phase über Leitung 11 von oben entgegengeführt (Einspeisung über einen Vorwärmer 8 mittels einer Hochdruckpumpe am Reaktorkopf), tauscht sich partiell im Sumpf aus, wird am Seitenabzug 2 gesammelt und nach der Bilanzierung zurückgeführt. Das gemäß dem Partialdruck ausgetragene Kopfwasser wird bedarfsweise durch frische Nitritlösung ersetzt.

Vorzugsweise ist zur Entfernung des restlichen Sauerstoffs bzw. Peroxoverbindungen ein Hydrierreaktor (nicht gezeigt) vorgeschaltet, der aus einem senkrecht stehenden Rohr mit Thermotasche mit einem Palladium- und/oder Platinkatalysator auf Trägermaterial bestehen kann. Der Reaktor ist z. B. für einen Betriebsdruck von ca. 10 bar ausgelegt. Der zu säubernde $C_4$-Schnitt wird mit Wasserstoff flüssig in den Hydrierreaktor eingespeist. Die $C_4$-Kohlenwasserstoffe durchströmen von unten nach oben ohne zusätzliche Erwärmung den Reaktor und werden dann anschließend in dem Gegenstromreaktor der eigentlichen thermischen Dimerisierung zugeführt.

Der Vorreaktor besteht z. B. aus einem senkrecht stehenden temperierbaren Rohr mit Ummantelung und einer Thermotasche und ist mit einem Palladium- und/oder Platinkatalysator auf Träger gefüllt. Wie schon erwähnt, können handelsübliche Hydrierkatalysatoren verwandt werden.

Die folgenden Beispiele dienen der Erläuterung der Erfindung.

## Beispiel 1

Als Dimerisierungsreaktor wurde ein senkrecht stehender Rohrreaktor mit 2,6 cm lichter Weite und einer Gesamtlänge von 150 cm benutzt. Der Nitritabzug erfolgte 50 cm oberhalb des Reaktorbodens. Die Temperaturmessung erfolgte mittels eines Thermoelementes, das in einer zentrisch angeordneten Thermometertasche von 0,6 cm ∅ verschiebbar angeordnet war. Das Reaktionsrohr war ummantelt und wurde mittels eines Wärmeübertragungsöles, Texatherm[R], indirekt beheizt. Der Reaktor wurde bis zum Niveauabzug mit einer ca. 1 molaren Natriumnitritlösung (6,9 Gew.% Natriumnitrit) geflutet. Am Reaktorboden wurden stündlich 140 g Pyrolyse-$C_4$ ex Raffinerie Heide, wenig belastet (ca. 71 Nl/h), mit einem Butadiengehalt von 35 Gew.% eingespeist. Gleichzeitig wurden am Reaktorkopf 0,5 l 6,9 %ige Natriumnitritlösung (1 l/l · h) zudosiert. Dieselbe Nitritmenge wurde kontinuierlich am Niveauabzug abgezogen. Für den gesamten Nitritkreislauf wurden 10 l Inhibitorlösung benutzt. Reaktorinnentemperatur betrug 200 °C, der Reaktionsdruck 150 bar.

Reaktionsprodukt, verdampftes Wasser und Restgas wurden am Reaktorkopf abgenommen. Im Hochdruckwasserabscheider 5 wurden stündlich etwa 4 g Wasser abgetrennt. Nach einstufiger Entspannung wurden die organischen Produkte im Gas-Flüssigkeitsscheider 7 aufgefangen und stabilisiert.

Es fielen stündlich 24 g stabilisiertes Dimerisat und 116 g Restgas an. Das Restgas enthielt durchschnittlich 1 Gew.% Vinylcyclohexen.

Zusammensetzung des stabilisierten Rohdimerisats :

| | |
|---|---|
| Gas | 0,2 Gew.% |
| Divinylcyclobutan | 1,4 Gew.% |
| Vinylcyclohexen | 84,8 Gew.% |
| Cyclooctadien-1,5 | 7,2 Gew.% |
| Unbekannte | 4,8 Gew.% |
| lösliche Oligomere | 1,6 Gew.% (Kp 100 °C/14 Torr) |

Der Gesamtumsatz des Butadiens/pass betrug somit 51 Gew.%.

Alle 250 Betriebsstunden wurde das Inhibitorvolumen durch 1 l frische Nitritlösung ergänzt. Nach 1 500 Betriebsstunden wurde der Reaktor geöffnet. Im Reaktor konnte kein festes Polymerisat (Popcorn) gefunden werden.

## Beispiel 2

Der Reaktor wurde gemäß Beispiel 1 mit 160 g reinem Butadien und 0,5 l 6,9 %iger Natriumnitritlösung belastet. Die Temperatur betrug 200 °C und der Gesamtdruck 150 bar. Man erhielt stündlich 118 g stabilisiertes Dimerisat, 42 g Restgas und 5 g Wasser. Das Dimerisat enthielt 0,1 % Butadien, 1,6 % Divinylcyclobuten, 86,7 % Vinylcyclohexen, 6,5 % Cyclooctadien, 3,9 % Trimere und 1,2 % lösliche Oligomere.

Der Butadienumsatz betrug 73,8 %/pass.

Nach 350 Betriebsstunden wurden keine Polymere gefunden.

## Beispiel 3

Der Reaktor wurde gemäß Beispiel 1 mit 150 g Pyrolyse-$C_4$ (Butadiengehalt 49,3 Gew.%) — mit einem Acetylengehalt unter 0,2 Gew.% — und 0,5 l 6,9 %iger Natriumnitritlösung belastet. Die Temperatur betrug 240 °C und der Druck 150 bar. Man erhielt stündlich 66,1 g Dimerisat, 83,9 g Restgas und 4 g Wasser. Das Dimerisat enthielt 0,1 % Gas, 0,2 % Divinylcyclobuten, 81,7 % Vinycyclohexen, 3,6 % Trimere und 6,0 % Unbekannte.

Der Butadienumsatz betrug 89,4 %/pass.

Nach 240 Betriebsstunden wurde der Reaktor geöffnet. Es konnte kein Polymerisat festgestellt werden.

## Beispiel 4

Der Reaktor wurde gemäß Beispiel 1 mit 150 g Pyrolyse-$C_4$ mit einem Butadiengehalt von 48,8 Gew.% und einem Acetylengehalt von 0,9 Gew.% belastet. Am Reaktorkopf wurden 0,5 l 6,9 %iger Natriumnitritlösung zudosiert. Die Temperatur betrug 240 °C und der Druck 190 bar. Man erhielt stündlich 67,4 g Dimerisat, 82,6 g Restgas und 4 g Wasser. Das Dimerisat enthielt 0,4 % Gas, 80,4 % Vinylcyclohexen, 4,2 % Äthylcyclohexen, 0,5 % Vinylcyclohexadien, 8,2 % Cyclooctadien, 3,7 % Trimere und 2,6 % Oligomere.

Der Butadienumsatz betrug 91,2 %/pass.

## Beispiel 5

Im Gegensatz zu Beispiel 1 wurden am Reaktorkopf sowohl 0,5 l 6,9 %iger Natriumnitritlösung (1 l/l · h) als auch 150 g Pyrolyse-$C_4$ (ca. 76 Nl/h) mit einem Butadiengehalt von 49,5 Gew.% eingespeist.

Nach Abkühlen und Abscheiden der organischen Phase wurde die anorganische Phase im Nitritkreislauf wieder zugeführt und die organische Phase nach einstufiger Entspannung im Gas/Flüssigkeitsscheider 7 aufgefangen und stabilisiert. Der Rohrreaktor wurde ansonsten gemäß Beispiel 1 betrieben.

Es wurden 59,8 g Dimerisat, 90,2 g Restgas erhalten. Das Dimerisat enthielt 0,2 % Gas, 0,9 % Divinylcyclobuten, 81,5 % Vinylcyclohexen, 2,2 % Äthylcyclohexen, 0,8 % Vinylcyclohexadien, 7,3 % Cyclooctadien, 3,2 % Trimere und 3,9 % Unbekannte.

Nach 400 Betriebsstunden wurde der Reaktor geöffnet. Es konnte kein Polymerisat (Popcorn) gefunden werden.

Der Butadienumsatz betrug 80,5 %/pass.

## Beispiel 6

Der Reaktor nach Beispiel 1 wurde gemäß Beispiel 1 mit 150 g Pyrolyse-$C_4$ (ca. 76 Nl/h) mit einem Butadiengehalt von 35 Gew.% belastet. Der Vorreaktor war mit einem Palladiumkatalysator auf $Al_2O_3$ gefüllt ; als Inertgas wurde $H_2$ benutzt. Gleichzeitig wurden am Reaktorkopf 0,5 l 6,9 %ige Natriumnitritlösung (1 l/l · h) zudosiert. Die Reaktorinnentemperatur betrug 240 °C, der Reaktionsdruck 190 bar. Man erhielt stündlich 33,6 g Dimerisat, 116,4 g Restgas. Das Dimerisat enthielt 0,1 % Gas, 0,8 % Divinylcyclobuten, 80,4 % Vinylcyclohexen, 2,8 % Äthylcyclohexen, 0,5 % Vinylcyclohexadien, 8,2 % Cyclooctadien, 5,3 % Unbekannte und 1,9 % Oligomere.

Der Butadienumsatz betrug 64 %/pass.

Alle 250 Betriebsstunden wurde das Inhibitorvolumen durch 1 l frische Nitritlösung ergänzt. Nach 1 500 Betriebsstunden wurde der Reaktor geöffnet. Im Reaktor konnte kein festes Polymerisat (Popcorn) gefunden werden.

## Vergleichsbeispiel 1

Es wurde ein Versuch gemäß Beispiel 1 durchgeführt, wobei anstelle der Inhibitorlösung dem Reaktor entsalztes Wasser zugeführt wurde. Belastungen, Temperatur- und Druckbedingungen waren dieselben wie im Beispiel 1. Nach 4 Stunden mußte der Versuch abgebrochen werden, da das eingespeiste Wasser über Kopf austrat und sich ein Differenzdruck von mehr als 10 bar aufgebaut hatte. Nach Öffnen des Reaktors wurden ca. 50 g Polymerisat gefunden.

Die Dimerisat-Zusammensetzung entsprach Beispiel 1.

## Vergleichsbeispiel 2

Der im Beispiel 1 beschriebene Reaktor wurde mit 250 ml einer 6,9 %igen Natriumnitritlösung gefüllt und auf 200 °C aufgeheizt. Am Fuße des Reaktors wurden stündlich 140 g Pyrolyse-$C_4$ mit 35 Gew.% Butadien zugeführt und über Kopf mit dem gebildeten Dimerisat derart abgezogen, daß ein Gesamtdruck von 150 bar resultierte. Am Kopf des Reaktors wurde nur das am Wasserabscheider anfallende Wasser zurückgeführt. Produktausbeute und Gaszusammensetzung entsprachen Beispiel 1. Nach 8 Stunden hatte sich ein Differenzdruck von 15 bar aufgebaut. bei der Inspektion des geöffneten Reaktors wurden oberhalb des Nitritniveaus ca. 35 g Polymerisat gefunden.

Das Vergleichsbeispiel 2 verdeutlicht, daß allein die Gegenwart von Nitrit für eine wirksame Inhibierung nicht ausreichend ist. Wesentlich ist, daß die Alkalinitritlösung am Kopf des Reaktors eingespeist wird.

0 053 293

Das Verfahren zur thermischen Dimerisierung von konjugierten Diolefinen dient sowohl zur Isolierung des Diolefins aus Kohlenwasserstoffgemischen durch Zersetzung des hergestellten Dimerisats als auch zur Herstellung des Dimerisats als chemisches Zwischenprodukt. So dient das durch Dimerisierung von Butadien hergestellte Vinylcyclohexen über Äthylbenzol der Herstellung von Styrol.

**Ansprüche**

1. Verfahren zur Herstellung von Vinylcyclohexen durch thermische Dimerisierung von Butadien in Gegenwart eines Polymerisationsinhibitors, dadurch gekennzeichnet, daß Butadien als solches oder als Bestandteil eines Kohlenwasserstoffschnittes bei einer Temperatur im Bereich von 180-260 °C bei erhöhtem Druck im Bereich von 50 bis 250 bar in Gegenwart einer wäßrigen Alkalinitritlösung in einem sich in vertikaler Richtung erstreckenden Reaktor dimerisiert wird, wobei die Alkalinitritlösung am Kopf des Reaktors eingespeist wird.

2. Verfahren nach Anspruch 1, dadurch gekennzeichnet, daß frisch destilliertes und/oder vorzugsweise mit Wasserstoff in Gegenwart eines Hydrierkatalysators behandeltes butadienhaltiges Material als Ausgangsmaterial eingesetzt wird.

3. Verfahren nach Anspruch 1 oder Anspruch 2, dadurch gekennzeichnet, daß die Dimerisierung in einem senkrecht stehenden Rohr-Reaktor durchgeführt wird.

4. Verfahren nach einem der Ansprüche 1 bis 3, dadurch gekennzeichnet, daß die Dimerisierung im Gegenstrom mit der Alkalinitritlösung durchgeführt wird.

5. Verfahren nach der vorhergehenden Ansprüche, dadurch gekennzeichnet, daß die Dimerisierung mit einer 4-40 gew.%igen wäßrigen Nitritlösung, durchgeführt wird.

6. Verfahren nach einem der vorhergehenden Ansprüche, dadurch gekennzeichnet, daß in Gegenwart von Natriumnitritlösung dimerisiert wird.

**Claims**

1. Process for producing vinylcyclohexene by thermal dimerization of butadiene in the presence of a polymerization inhibitor, characterized by dimerizing butadiene as such or as a constituent of a hydrocarbons cut at a temperature of 180 to 260 °C, an elevated pressure of 50 to 250 bar, and in the presence of an aqueous alkali nitrite solution in a vertically extended reactor, the alkali nitrite solution being charged to the reactor head.

2. Process according to claim 1, characterized by using as a starting material a butadiene-containing feedstock that has been freshly distilled and/or treated preferably with hydrogen in the presence of a hydrogenating catalyst.

3. Process according to claim 1 or 2, characterized by performing the dimerization in an upright tube reactor.

4. Process according to any one of claims 1 through 3, characterized by performing the dimerization in countercurrent operation with the alkali nitrite solution.

5. Process according to any one of the preceding claims, characterized by performing the dimerization with a 4-40 %wt. aqueous nitrite solution, preferably with a 4-9 %wt. aqueous nitrite solution.

6. Process according to any one of the preceding claims, characterized by performing the dimerization in the presence of sodium nitrite solution.

**Revendications**

1. Procédé de préparation de vinylcyclohexène par dimérisation thermique de butadiène en présence d'un inhibiteur de polymérisation, caractérisé en ce que le butadiène tel quel ou en tant que constituant d'une coupe d'hydrocarbures est dimérisé à une température de 180 à 260 °C, à une pression élevée de 50 à 250 bars en présence d'une solution aqueuse d'alcali dans un réacteur vertical, la solution de nitrite d'alcali étant alimentée à la tête du réacteur.

2. Procédé selon la revendication 1, caractérisé en ce qu'on utilise comme produit de départ une matière contenant du butadiène, cette matière étant fraîche et/ou ayant été traitée préférablement d'hydrogène en présence d'un catalyseur d'hydrogénation.

3. Procédé selon la revendication 1 ou 2, caractérisé en ce que la dimérisation est effectuée dans un réacteur à tube vertical.

4. Procédé selon l'une des revendications 1 à 3, caractérisé en ce que la dimérisation est effectuée en chargeant la solution de nitrite d'alcali en contre-courant.

5. Procédé selon l'une des revendications précédentes, caractérisé en ce que la dimérisation est effectuée avec 4 à 40 % en poids, de préférence 4 à 9 % en poids, de solution aqueuse de nitrite.

6. Procédé selon l'une des revendications précédentes, caractérisé en ce que la dimérisation est effectuée en présence d'une solution de nitrite de sodium.

0 053 293